# EUROPÄISCHE PATENTSCHRIFT

(11) **EP 3 773 389 B1**
(45) Veröffentlichungstag und Bekanntmachung des Hinweises auf die Patenterteilung: **17.07.2024**
(21) Anmeldenummer: 19721741.7
(22) Anmeldetag: 20.03.2019
(51) Int. Cl.: A61F 13/20

(54) **VORRICHTUNG ZUR FORMUNG VON TAMPONS**
DEVICE FOR SHAPING TAMPONS
DISPOSITIF DE FORMATION DE TAMPONS

(30) Priorität: 29.03.2018 CH 4262018
(43) Veröffentlichungstag der Anmeldung: 17.02.2021
(73) Patentinhaber: Ruggli AG, 5322 Koblenz (CH)
(72) Erfinder: SCHULER, Samuel, 4051 Basel (CH); AUER, Marco, 79761 Waldshut-Tiengen (DE)
(74) Vertreter: IPrime Rentsch Kaelin AG
(86) Internationale Anmeldenummer: PCT/IB2019/052261
(87) Internationale Veröffentlichungsnummer: WO 2019/186325

(56) Entgegenhaltungen:
- EP-A1- 2 412 351
- US-A- 2 566 190

## Beschreibung

Die vorliegende Erfindung betrifft eine Vorrichtung zur Formung von Tampons aus bandförmigem Material, sowie ein Verfahren zur Formung eines Tampons aus bandförmigem Material, beides gemäss Oberbegriff der kennzeichnenden Ansprüche.

### Technologischer Hintergrund

Für die weibliche Hygiene insbesondere während der Regelblutung verwendete Tampons bestehen im Wesentlichen aus einem saugfähigen Material, meistens einem Viskose-Wattestreifen, welches zur charakteristischen, länglichen Form zunächst gewickelt, dann verpresst und geformt wird. Diese Verarbeitungsprozesse vom bandförmigen Ausgangsmaterial zum fertigen Tampon werden schrittweise in getakteten Fertigungsstrassen durchgeführt. In einem ersten Schritt wird zunächst der Streifen des Materials gewickelt, wobei ein Rückholfaden um den Streifen gelegt wird und vor dem Wickeln verknotet wird. Anschliessend wird der Wickel mit Druck verpresst. Durch eine entsprechende Ausgestaltung des Presswerkzeugs ist es möglich, bestimmte Strukturen am fertigen Tampon zu erzeugen, wie z.B. die gängigen Längsrillen. In den meisten Herstellungsverfahren wird zusätzlich, um einen besonders glatten und runden Tamponkopf zu erhalten, ein Kopfformschritt durchgeführt, welcher den Tamponkopf zusätzlich formt und/oder glättet. Jeder dieser Bearbeitungsschritte findet an einer spezialisierten Arbeitsstation statt und setzt einen getakteten Transfer von einer Arbeitsstation zur nächsten voraus.

Durch den Takt dieser Übergabe sind einer Tamponfertigungsstrasse Grenzen bzgl. der möglichen erreichbaren Maximalgeschwindigkeit gesetzt. Das dauernde Beschleunigen und Bremsen der Betriebseinheiten führt zudem zu einem starken Verschleiss und hohen Energieverbrauch.

Besondere Beachtung bedarf bei der Tamponproduktion auch die Empfindlichkeit der bearbeiteten Materialien, da die Watte, resp. das Watte/Vliesband, mit entsprechender Vorsicht befördert werden muss, wenn nicht Qualitätseinbussen am Endprodukt in Kauf zu nehmen sind. Weiter erschwerend kommt die stark hygroskopische Eigenschaft des Materials hinzu, wobei die Luftfeuchtigkeit in einem engen Rahmen gehalten werden muss, da eine optimale Materialfeuchtigkeit erst den sicheren Prozessablauf gewährleistet.

Es besteht somit ein Bedarf nach Vorrichtungen und Verfahren zur Formung von Tampons, welche eine hohe Prozessgeschwindigkeit erreichen können, ohne dabei Nachteile in der Qualität der erzeugten Produkte in Kauf zu nehmen.

Dabei misst sich bei der Produktion von Tampons die Geschwindigkeit in der Anzahl verarbeiteter Werkstücke pro Minute. Diese Stückzahlen liegen in der gegenwärtigen Technik bei zwischen 100 und 140 Stück pro Minute maximal, bewegen sich jedoch oft real darunter.

### Darstellung der Erfindung

Somit ist es eine Aufgabe der vorliegenden Erfindung, eine Vorrichtung zur Herstellung von Tampons bereitzustellen, welche mindestens eine der oben genannten Probleme überwindet. Insbesondere soll eine Vorrichtung zur Herstellung von Tampons bereitgestellt werden, welche Stückzahlen von über 140 Stück pro Minute zu erreichen vermag. Entsprechend soll auch ein Verfahren bereitgestellt werden, welches mindestens einen der Nachteile des Bekannten überwindet, insbesondere Stückzahlen von über 140 Stück pro Minute zu verarbeiten vermag.

Diese Aufgabe wird mit einer Vorrichtung zur Formung von Tampons, wie im kennzeichnenden Teil der unabhängigen Ansprüche beschrieben, gelöst.

Ein Aspekt der vorliegenden Erfindung betrifft somit eine Vorrichtung zur Formung von Tampons aus einem bandförmigen Material. Diese Vorrichtung umfasst eine Mehrzahl an Bearbeitungstrommeln und mindestens eine Übergabestation, ein Übergaberad, zur kontinuierlichen Übergabe eines Vorformlings von einer ersten Bearbeitungstrommel an eine zweite Bearbeitungstrommel. Somit ist eine kontinuierlich betreibbare Vorrichtung zur Herstellung eines Tampons oder Vorformlings bereitgestellt.

Für die vorliegende Erfindung wird von einem bandförmigen Material ausgegangen, welches bereits vorgängige Verarbeitungsschritte durchlaufen haben kann. So sind insbesondere bandförmige Materialien für die Tamponproduktion in Verwendung, welche aus laminierten Schichten von gewobenen und/oder nicht gewobenen Materialien bestehen. Gängigerweise wird ein Vlies-/Wattematerial verwendet. Für die Erfindung ist die Zusammensetzung dieser bandförmigen Materialien im Detail von untergeordneter Bedeutung.

Im Sinne der vorliegenden Erfindung kann unter einem Tampon jede Art von zu einem in Wesentlichen länglichen Objekt gepressten Wattestab verstanden werden, welcher hauptsächlich dazu dient in eine Körperöffnung, oder eine Wunde geführt zu werden. Die Formung umfasst im Sinne der Erfindung die erforderlichen Schritte, um aus dem bandförmigen Material einen eben solchen Tampon zu machen, insbesondere einen Tampon, der eine sich an einem Kopfende im Wesentlichen verjüngende Kopfform und einen länglichen Körper aufweist. Solche Tampons werden in der Regel als Hygieneprodukte von Frauen während der Regelblutung verwendet. Tampons, die anderen Verwendungszwecken zugeführt werden, können aber gleichwohl mit der erfindungsgemässen Vorrichtung hergestellt werden. So können auch Analtampons oder hämostatische Tampons für den chirurgischen Gebrauch mit der erfindungsgemässen Vorrichtung ohne, oder mit lediglich geringfügigen Anpassungen hergestellt werden. Solche Tampons können zum Beispiel ohne verjüngenden Kopf ausgestaltet sein.

Der erfindungsgemässen Vorrichtung nachgelagert, können noch weitere Verarbeitungsschritte folgen, wie z.B. das Verpacken der Tampons in eine Folie oder einen Applikator. In einer besonderen Ausführungsform können das Verpacken der Tampons in eine Folie oder einen Applikator durch erfindungsgemässe Bauteile ausgeführt werden, zum Beispiel durch Bearbeitungstrommeln. In einer weiteren besonderen Ausführungsform ist für das Verpacken eine Verpackungstrommel vorgesehen.

Die CA 2085923, zum Beispiel, zeigt ein Verfahren, wie ein bandförmiges Material, welches für die Vorrichtung der vorliegenden Erfindung geeignet wäre, vorbereitet werden kann. EP2412351 zeigt eine Mehrzahl an Bearbeitungstrommeln. Die Übergabe findet direkt zwischen den Bearbeitungstrommeln statt.

Im Sinne der vorliegenden Erfindung kann als Bearbeitungstrommel eine um eine zentrale Achse rotierbar gelagerte Trommel verstanden werden, welche radial angeordnete Bearbeitungswerkzeuge für einen spezifischen Formungsschritt der Tamponformung aufweist. Die Bearbeitungstrommel kann direkt angetrieben sein, insbesondere kann jede Bearbeitungstrommel separat direkt angetrieben sein.

Im Sinne der vorliegenden Erfindung kann als eine Übergabestation eine zwischen den Bearbeitungstrommeln positionierbare Station mit radial umlaufend angeordneten Aufnahmegreifern zur kontinuierlichen Übergabe eines Werkstücks verstanden werden. Dazu können zum Beispiel umlaufende Riemen, Bänder, Gurte oder Ketten, insbesondere Kettenbänder, vorgesehen sein, welche umlaufend angeordnete Aufnahmegreifer aufweisen, insbesondere welche entlang eines Verlaufs des Aussenumfangs eines Riemens oder Bandes eine Mehrzahl an Aufnahmegreifer umfassen. Durch solche Übergabestationen kann die Übergabe der Werkstücke stetig und kontinuierlich erfolgen.

In einer besonderen Ausführungsform ist die Übergabestation ein Übergaberad mit radial umlaufend angeordneten Aufnahmegreifern, welche bei der Rotation um die Rotationsachse des Übergaberades einen im Wesentlichen kreisförmigen Radius beschreiben.

In einer besonderen Ausführungsform umfasst die erfindungsgemässe Vorrichtung mindestens eine erste und mindestens eine zweite Bearbeitungstrommel. Eine erste Bearbeitungstrommel kann ausgelegt sein, das zugeführte bandförmige Material zu einem Wickel zu wickeln. Die zweite Bearbeitungstrommel kann dazu vorgesehen sein, den besagten Wickel zu verpressen.

Im Sinne der vorliegenden Erfindung wird der Begriff Vorformling für ein Vorläuferprodukt eines Tampons verwendet, welcher mindestens einen Bearbeitungsschritt durchlaufen hat. So kann das gewickelte bandförmige Material im Sinne der vorliegenden Erfindung als der erste Vorformling angesehen werden. Wird dieser Wickel, oder Vorformling gepresst, so kann das resultierende Produkt bereits ein Tampon sein, oder, falls es durch weitere Schritte bearbeitet wird, zu einem zweiten Vorformling werden, der wiederum weiterverarbeitet werden kann zu einem Tampon oder evtl. sogar einem dritten Vorformling. So können z.B. nach dem Verpressen weitere qualitätssteigernde Bearbeitungsschritte vorgesehen sein, welche an den Vorformlingen durchgeführt werden.

In einer besonderen Ausführungsform umfasst die vorliegende Erfindung eine dritte Bearbeitungstrommel, welche eine Kopfformung und/oder Glättung des zweiten Vorformlings durchführt.

In einer weiteren besonderen Ausführungsform umfasst die erfindungsgemässe Vorrichtung eine vierte Bearbeitungstrommel, welche ausgelegt ist eine Glättung des Tamponkopfes oder der Tamponoberfläche durchzuführen.

In einer besonderen Ausführungsform sind die Bearbeitungstrommeln in einer Fertigungsstrasse hintereinander angeordnet, so dass jeder Prozessschritt in einer bestimmten Reihenfolge von den Vorformlingen durchgangen wird. Besonders bevorzugt ist zwischen jedem Paar an benachbarten Bearbeitungstrommeln jeweils eine Übergabestation angeordnet, welche für die Übergabe der Vorformlinge zwischen den Bearbeitungstrommeln verantwortlich ist.

Die Übergabestation ist so in der erfindungsgemässen Vorrichtung angeordnet, dass die Übergabe eines Vorformlings von einer ersten Bearbeitungstrommel an eine zweite Bearbeitungstrommel kontinuierlich ist. Bevorzugt ist die Übergabestation ein Übergaberad.

Bisherige Verfahren und Vorrichtungen zur Herstellung und Formung von Tampons laufen insgesamt diskontinuierlich ab, weil mindestens eine Übergabe zwischen den Bearbeitungsgeräten über eine intermittierend operierende Übergabeeinheit stattfindet. Mit der erfindungsgemässen Vorrichtung ist es möglich, in einem kontinuierlichen Endlos-Verfahren Tampons mit hohen Stückzahlen pro Minute herzustellen.

Dadurch, dass die Übergabe über ein Übergaberad erfolgt, wird zudem der Wartungsaufwand reduziert, indem weniger Verschleiss an den ansonsten in einem Stop-and-go-Verfahren operierenden Bauteilen stattfindet. Der Maschinenbetrieb ist insgesamt ruhiger und der Verschleiss reduziert.

Ein weiterer Vorteil der erfindungsgemässen Vorrichtung ist, dass der kontinuierlich ablaufende Prozess skalierbar ist. So kann zum Beispiel die Anzahl der Werkzeuge an den Bearbeitungstrommeln erhöht werden, wodurch die Raumnutzung in Fertigungsanlagen optimiert werden kann. Obschon Fertigungsanlagen mit mehr Werkzeugen pro Bearbeitungstrommeln voluminöser ausfallen können, ist der Raumzuwachs kleiner, als mehrere Fertigungsstrassen parallel aufzustellen. Die Skalierbarkeit ermöglicht es auch spezifisch auf Kundenwünsche zugeschnittene Fertigungsstrassen zu entwerfen, welche eine gewünschte Anzahl an Bearbeitungswerkzeugen pro Bearbeitungstrommel aufweisen. Gleichzeitig kann die Bearbeitungsdauer am Werkstück für die einzelnen Bearbeitungswerkzeuge konstant gehalten werden. Etwaige Differenzen in der Anzahl und/oder Bearbeitungsgeschwindigkeit zwischen zwei benachbarten Bearbeitungstrommeln können durch die kontinuierlich arbeitenden Übergabestationen, respektive Übergaberäder aufgefangen werden.

Für gewisse Anwendungen kann es sinnvoll sein, die Stückzahl unter die maximal mögliche Ausstosszahl zu senken. Dies kann z.B. bei Kontroll- oder Eichungsprozessen der Vorrichtungen von Nutzen sein, aber auch, um entsprechende nachbearbeitende Maschinen zu akkommodieren. Durch die stufenlose Skalierbarkeit des kontinuierlichen betreibbaren Übergaberades wird auch dies ermöglicht. Trotz der hohen möglichen Prozessgeschwindigkeit und Stückzahlen, kann somit durch die erfindungsgemässe Vorrichtung mehr Zeit für die einzelnen Prozessschritte bleiben. So kann, zum Beispiel, das Werkstück während der gesamten Verweildauer auf einer Bearbeitungstrommel im Werkzeug verbleiben und wird gleichzeitig in Förderrichtung befördert. Mehrere Werkzeuge pro Bearbeitungstrommel ermöglichen höhere Stückzahlen, ohne dabei die Bearbeitungszeit pro Werkstück zu senken. So kann zum Beispiel die zum Pressen ausgestaltete Bearbeitungstrommel über eine Mehrzahl an Bearbeitungswerkzeugen, also Pressen verfügen, bevorzugt sechs Pressen, die jeweils für einen Zeitraum von der Übernahme des Werkstücks von einem Übergaberad, bis zur Übergabe an ein weiteres Übergaberad das Werkstück bearbeiten können. Die radial nächste Rolle übernimmt derweil das nächste Werkstück.

In einer besonderen Ausführungsform umfasst eine Bearbeitungstrommel mindestens zwei radial angeordnete Bearbeitungswerkzeuge. Bevorzugt werden die Bearbeitungswerkzeuge so angeordnet, dass bei einem Betrieb entstehende Fliehkräfte auf den Aussenradien der Bearbeitungstrommeln ausgeglichen werden, d.h. die Bearbeitungswerkzeuge sind um die zentrale Rotationsachse symmetrisch zueinander angeordnet.

In einer besonderen Ausführungsform umfasst eine Bearbeitungstrommel zwischen sechs und vierundzwanzig Bearbeitungswerkzeuge.

Hier offenbart sich ein weiterer Vorteil der vorliegenden Erfindung. Durch die stufenlos betreibbaren und kontinuierlich arbeitenden Übergaberäder ist es möglich, zwischen zwei Bearbeitungstrommeln mit unterschiedlicher Anzahl an Bearbeitungswerkzeugen dennoch eine kontinuierliche Zufuhr an Werkstücken zu ermöglichen.

In einer besonderen Ausführungsform ist jedes Übergaberad der erfindungsgemässen Vorrichtung separat steuerbar.

In einer besonderen Ausführungsform sind die Bearbeitungstrommeln so ausgestaltet, dass sie während der rotatorischen Bewegung um ihre Trommelachse das Werkstück, oder den Vorformling bearbeiten und in eine Förderrichtung der Vorrichtung befördern. Mit anderen Worten kann auch die Bearbeitung durch die Bearbeitungstrommel vorzugsweise kontinuierlich ausgestaltet sein.

In einer besonderen Ausführungsform umfasst das Übergaberad eine Mehrzahl an Aufnahmegreifern. Diese Aufnahmegreifer sind so ausgelegt, dass sie in einen Übergabebereich an einem Rotationsradius mindestens einer der Bearbeitungstrommeln eingreifen. Im Betrieb rotieren die Bearbeitungstrommeln um ihre Trommelachse, sodass sich ein äusserer Radius bildet, auf dem die Bearbeitungswerkzeuge auf der Bearbeitungstrommel angeordnet sind. Bevorzugt wird in der vorliegenden Vorrichtung eine Bearbeitungstrommel und ein Übergaberad so zueinander positioniert, dass ein Übergabebereich entsteht. Aus diesem Übergabebereich kann ein Übergaberad ein entsprechendes von der Bearbeitungstrommel fertig bearbeitetes Werkstück oder Vorformling aufgreifen und an die nächste Bearbeitungstrommel übergeben.

In einer besonderen Ausführungsform umfasst das Übergaberad mindestens eine Führungskurve. Diese Führungskurve kann die Aufnahmegreifer auf einer Radialbewegung des Übergaberads so führen, dass bei einer Rotation des Übergaberads relativ zu einer ersten Bewegungstrommel ein Übergabebereich definiert wird, in welchem ein Aufnahmenest eines Aufnahmegreifers im Wesentlichen koaxial zu einem Bearbeitungswerkzeug einer Bearbeitungstrommel steht.

Im Sinne der vorliegenden Erfindung stehen das Aufnahmenest und das Bearbeitungswerkzeug koaxial zueinander, wenn sie bezüglich ihrer Längsachse koaxial angeordnet sind. Diese Längsachse kann auch der Längsachse des Werkstücks im Bearbeitungswerkzeug oder Aufnahmenest entsprechen.

In einer besonderen Ausführungsform der vorliegenden Erfindung weist das Übergaberad radial angeordnete Aufnahmegreifer auf. Diese Aufnahmegreifer umfassen ein Aufnahmenest zur Aufnahme eines Tampons oder eines Vorformlings. Sie umfassen weiter eine Führungsstange, an der das Aufnahmenest angeordnet ist. Zudem umfasst der erfindungsgemässe Aufnahmegreifer eine Führungsbuchse, durch welche die Führungsstange in ihrer Längsachse verschiebbar gelagert ist. Diese Führungsbuchse kann wiederum an einer rotierbaren Drehscheibe befestigt sein.

In einer besonderen Ausführungsform ist die Führungsbuchse schwenkbar auf der rotierbaren Drehscheibe um eine Rotationsachse gelagert. Zum Beispiel kann die Führungsbuchse mittels eines Radiallagers schwenkbar zur Drehscheibe gelagert sein. Bevorzugt ist diese Rotationsachse parallel zur Rotationsachse des Übergaberades, also rechtwinklig zur Längsachse der Führungsstange ausgestaltet ist. Ein Vorteil dieser Anordnung ist, dass für die Aufnahmegreifer entlang ihrer Führungskurven eine Geschwindigkeitskorrektur ihrer Tangentialgeschwindigkeit vorgenommen werden kann.

Zusätzlich zu einer Längsverschiebung der Führungsstange entlang des Lagers der Führungsbuchse ist nun der gesamte Aufnahmegreifer um eine Drehachse schwenkbar. Dies ermöglicht einen weiteren Freiheitsgrad und ein Anpassen der Kurvengeschwindigkeit des Aufnahmenests an die Kurvengeschwindigkeit des Werkzeugs der Bearbeitungstrommel im Übergabebereich. Das Aufnahmenest kann im Übergabebereich eine nicht konstante Geschwindigkeit entlang der Führungskurve abfahren.

In einer besonderen Ausführungsform umfasst die Führungsstange weiter ein erstes Eingreifmittel zur Wirkverbindung mit einer ersten Führungskurve. Im Betrieb wird das Übergaberad bei der Rotation der Drehscheibe parallel zu einer ersten starr angeordneten Führungskurve rotiert, welche als Kulisse für die einzelnen Aufnahmegreifer wirkt. Die Aufnahmegreifer können mittels eines als Zapfen oder Nocken ausgebildeten Eingreifmittels mit der Führungskurve in Wirkverbindung gebracht werden. Somit durchlaufen die Aufnahmegreifer nicht lediglich eine rotatorische Bewegung um die Rotationsachse des Übergaberads, sondern vollführen während dieser Bewegung auch eine Kurve, welche durch die Führungskurve definiert ist. Dadurch ist es möglich, im Übergabebereich zu einer Bearbeitungstrommel in diese einzugreifen und in eine koaxiale Lage zu einem Werkzeug der Bearbeitungstrommel zu gelangen, wobei für einen bestimmten Zeitpunkt eine im Wesentlichen gleiche Kurvengeschwindigkeit besteht, sodass ein Werkstück vom Werkzeug in das Aufnahmenest des Aufnahmegreifers übertragen werden kann.

In einer besonderen Ausführungsform umfasst der Aufnahmegreifer ein zweites Eingreifmittel zur Wirkverbindung mit einer zweiten Führungskurve. Das Übergaberad wird bei der Rotation der Drehscheibe parallel zur zweiten starr angeordneten Führungskurve rotiert, welche als Kulisse für die Führungsbuchse dient.

Der Aufnahmegreifer ist bevorzugt so ausgestaltet, dass das Aufnahmenest mindestens zwei Freiheitsgrade aufweist. Ein erster Freiheitsgrad wird durch Lagerung der Führungsstange in ihrer Längsachse an der Führungsbuchse erreicht. Ein weiterer Freiheitsgrad wird durch die Rotationslagerung der Führungsbuchse auf der Drehscheibe erreicht.

In einer besonderen Ausführungsform sind die erste Führungskurve und die zweite Führungskurve parallel angeordnet, insbesondere als Nuten auf zwei parallelen Führungsscheiben angeordnet. Die Nuten können unterschiedliche Kurvenbahnen ablaufen. Zwischen den beiden parallelen Führungsscheiben mit den Führungskurven sind die Aufnahmegreifer auf der Drehscheibe rotierbar gelagert angeordnet. Die Aufnahmegreifer weisen einen ersten Nocken zur Wirkverbindung mit der ersten Führungskurve, und einen zweiten Nocken zur Wirkverbindung mit der zweiten Führungskurve auf. Die Nocken sind auf den Aufnahmegreifern gegenüberliegend in dieser Ausführungsform angeordnet, bevorzugt aber nicht konzentrisch angeordnet.

Durch diese Anordnung wird der Aufnahmegreifer auf seiner Radialbewegung entlang einer Kurve so geführt, dass er mindestens zeitweise in einem Übergabereich die gleiche Kurvengeschwindigkeit wie ein radial angeordnetes Werkzeug einer Bearbeitungstrommel aufweist.

In einer besonderen Ausführungsform ist das Übergaberad als Trommel ausgestaltet, wobei die Aufnahmegreifer auf einer rotierbar zwischen zwei starren Führungsscheiben angeordneten Drehscheibe angeordnet sind.

In einer besonderen Ausführungsform umfassen die Bearbeitungstrommeln eine Ausstosstrommel, welche parallel zur Bearbeitungstrommel angeordnet ist und um die gleiche Achse wie die zugehörige Bearbeitungstrommel rotierbar angeordnet ist.

In einer besonderen Ausführungsform umfasst die Ausstosstrommel eine Mehrzahl an Ausstossstösseln, welche derart auf einem Radius der Ausstosstrommel angeordnet sind, dass sie parallel zu den Bearbeitungswerkzeugen der zugehörigen Bearbeitungstrommel angeordnet sind.

In einer weiteren bevorzugten Ausführungsform sind die Ausstossstössel so auf der Ausstosstrommel angeordnet, dass sie im Wesentlichen koaxial zu den Werkzeugen verlaufen. In dieser Ausführungsform wird im Übergabebereich bei der Übergabe von der Bearbeitungstrommel zum Übergaberad oder umgekehrt eine koaxiale Anordnung des Ausstossstössels einer Ausstosstrommel, des Werkzeugs ihrer zugehörigen Bearbeitungstrommel und des Aufnahmenests des Übergaberads stattfinden.

In einer weiteren besonderen Ausführungsform umfasst die Ausstosstrommel eine dreidimensionale Kulisse, entlang welcher die Ausstossstössel geführt sind. Die Kulisse kann so angeordnet sein, dass die Ausstossstössel in den Zwischenraum zwischen der Ausstosstrommel und der Bearbeitungstrommel wirken, wenn eine koaxiale Anordnung zu einem Aufnahmenest eines Übergaberads besteht. So kann z.B. die Übergabe eines Werkstücks oder Vorformlings von einem Übergaberad in ein entsprechendes Bearbeitungswerkzeug gewährleistet werden.

In einer besonderen Ausführungsform ist die Führungskurve als Nut ausgebildet, in die ein Zapfen oder Bolzen eines Aufnahmegreifers dergestalt eingreifen kann, dass bei einer Bewegung des Aufnahmegreifers um die Rotationsachse des Übergaberads eine Verschiebung der Führungsstange entlang der Führungsbuchse in Längsrichtung geschieht.

Alternativ zu einer Führungskurve können die Aufnahmegreifer auch aktuiert ausgestaltet sein. In diesem Fall würden elektrische, pneumatische oder hydraulische Aktuatoren eine Bewegung der Führungsstange in radialer Richtung bzgl. des Übergaberads ermöglichen. Entsprechende zusätzliche Aktuatoren könnten vorgesehen sein, um eine Schwenkbewegung des Übernahmegreifers zu ermöglichen.

In einer weiteren alternativen Ausführungsform können die Aufnahmegreifer auch federnd gelagert sein. In einer besonderen Ausführungsform dieser Variante umfasst eine Bearbeitungstrommel Mittel, um mit den Aufnahmegreifern zumindest auf einem Übergabebereich ihres Radius in Wirkverbindung zu treten. So können zum Beispiel Haken an der Bearbeitungstrommel vorgesehen sein, welche mit entsprechenden Ösen der Aufnahmegreifer in Wirkverbindung treten. Dieser Kontakt kann für die Dauer einer Werkstückübertragung, beispielsweise durch einen Ausstossstössel, aufrechterhalten werden. Alternativen oder Ergänzungen zu den Haken und Ösen wären magnetische Mitnehmer, welche elektrisch an- und ausstellbar sein können, oder mittels Ansaugung von Luft betriebene Mitnehmer. Im Sinne der vorliegenden Erfindung kann ein Aufnahmegreifer federnd gelagert werden, indem eine Feder angeordnet wird, dass sie eine Rückstellkraft auf ein Aufnahmenest in radialer Richtung auszuüben vermag.

In einer besonderen Ausführungsform sind die Ausstosstrommeln am Ort der Ausstossstössel zusätzlich mit Saugvorrichtungen ausgestattet, welche einen Luftstrom in Richtung der Werkzeuge der Bearbeitungstrommel aufrechterhalten. Dies dient dazu, Auszugsfäden der Tampons im Bearbeitungsbereich zu kontrollieren, was ein Verheddern der Tamponfäden verhindert. Zu diesem Zweck können die Aufnahmenester auch mit einer Aufnahmenut vorgesehen sein, welche es ermöglicht, dass die Aufnahmenester beim Eingriff in den Übergabebereich durch ein entsprechendes Saugelement der Ausstosstrommel horizontal in den Übergabebereich hineinragende Auszugsfäden nicht zu kontaktieren. Entsprechende Saugelemente können auch an den Übergaberädern vorgesehen sein, und zwar entsprechend für jedes Aufnahmenest, um bei der Übergabe ein Verheddern der Auszugsfäden zu verhindern.

In einer besonderen Ausführungsform handelt es sich bei der ersten Bearbeitungstrommel um eine Wickeltrommel. Bevorzugt weist die Wickeltrommel zwischen sechs und 24, besonders bevorzugt zwölf Werkzeuge auf.

In einer besonderen Ausführungsform ist hier die zweite Bearbeitungstrommel eine Bearbeitungstrommel mit einer Mehrzahl an Tamponpressen, bevorzugt zwischen zwei und zwölf Tamponpressen, besonders bevorzugt sechs Tamponpressen. Tamponpressen, welche ausgelegt sind, um radial über eine Mehrzahl an Pressbacken Druck auf einen Wickel auszuüben und somit zu einem Tampon zu verpressen, sind im Stand der Technik bekannt.

In einer besonderen Ausführungsform sind alle Bauteile, welche physischen Kontakt mit dem bandförmigen Material, einem Vorformling oder dem Tampon aufweisen, antistatisch beschichtet oder bestehen im Wesentlichen aus einem antistatischen Material.

In einer besonderen Ausführungsform umfasst die Vorrichtung für die Übergabestationen, insbesondere Übergaberäder und Bearbeitungstrommeln Antriebe.

Ein weiterer Aspekt der vorliegenden Erfindung betrifft ein Verfahren zur Formung eines Tampons aus bandförmigem Material. In einem ersten Schritt wird ein bandförmiges Material bereitgestellt. Das bandförmige Material kann dabei, wie eingangs geschildert, bereits vorverarbeitet worden sein und ist in den gängigen Fällen ein Laminat aus einem Vliesstoff und einer Watte. Das bandförmige Material wird schrittweise über eine Mehrzahl drehender Bearbeitungstrommeln geformt. Bevorzugt handelt es sich um kontinuierlich drehende Bearbeitungstrommeln. Dabei erfolgt eine Übergabe von einer ersten Bearbeitungstrommel zu einer zweiten Bearbeitungstrommel kontinuierlich mittels eines einer Übergabestation, insbesondere mittels eines Übergaberads.

Durch das erfindungsgemässe Verfahren wird die Stückzahlleistung einer Vorrichtung zur Formung von Tampons von der Bearbeitungszeit des einzelnen Werkstücks entkoppelt. Trotz der hohen möglichen Stückzahlen, müssen keine Einbussen bei Bearbeitungszeiträumen der einzelnen Werkstücke hingenommen werden. Dies ermöglicht eine Fertigung mit hoher Leistung, bei gleichzeitig sehr hoher Qualität.

In einer besonderen Ausführungsform des erfindungsgemässen Verfahrens umfasst das schrittweise Formen des bandförmigen Materials einen Wickelschritt, einen Pressschritt und einen Kopfform- und/oder Glättungsschritt. Dabei erfolgt jeder dieser Schritte auf einer bestimmten, kontinuierlich drehenden Bearbeitungstrommel.

In einer besonderen Ausführungsform bewegen die Bearbeitungstrommeln bei jedem schrittweisen Formen das Werkstück oder den Vorformling in eine Förderrichtung weiter.

In einer besonderen Ausführungsform vollführt die Übergabestation oder das Übergaberad bei der Übergabe von einer ersten Bearbeitungstrommel zu einer zweiten Bearbeitungstrommel ein Eingreifen mit einem Aufnahmegreifer in einen Übergabebereich einer ersten Bearbeitungstrommel, sodass zumindest zeitweise ein Aufnahmenest des Aufnahmegreifers sich in einer koaxialen Anordnung zu einem Werkzeug befindet. Eine Ausstosstrommel vollführt den Schritt des Ausstossens eines Werkstücks aus dem Werkzeug in das Aufnahmenest oder aus dem Aufnahmenest in das Werkzeug, insbesondere geschieht dies durch Verschieben eines Ausstossstössels in Längsrichtung des Werkzeugs oder Aufnahmenests.

Im Sinne der vorliegenden Erfindung ist von einer koaxialen Anordnung des Werkzeugs, des Aufnahmenests und des Ausstossstössels stets in Verbindung mit der Längsausdehnung der entsprechenden Elemente die Rede. Dies entspricht auch der Längsausdehnung der mit dem erfindungsgemässen Verfahren hergestellten Tampons, resp. der mit der erfindungsgemässen Vorrichtung geformten Tampons.

In einer besonderen Ausführungsform ist der Ausstossstössel im zweiten Schritt so auf einer Ausstosstrommel angeordnet, die parallel zur entsprechenden Bearbeitungstrommel rotiert, sodass die Ausstossstössel jeweils einem entsprechenden Werkzeug zugeordnet sind.

In einer weiteren besonderen Ausführungsform des erfindungsgemässen Verfahrens ist ein Aufnahmegreifer des Übergaberads bei der Rotation desselben über eine Führungskurve so geführt, dass in einem Übergabebereich zwischen der Bearbeitungstrommel und einem Aufnahmenest die gleiche Bahngeschwindigkeit besteht. Bevorzugt besteht diese gleiche Bahngeschwindigkeit zu einem Werkzeug der Bearbeitungstrommel.

Für einen Fachmann versteht es sich von selbst, dass sämtliche sich nicht gegenseitig ausschliessende Ausführungsformen der vorliegenden Erfindung in einer Ausgestaltung in einer beliebigen Kombination verwirklicht sein können.

### Figurenbeschrieb

Im Folgenden wird nun die vorliegende Erfindung anhand konkreter Ausführungsbeispiele und schematischer Zeichnungen weiter erläutert, ohne jedoch auf diese besonderen Ausführungsformen beschränkt zu sein.

Der Einfachheit halber werden in den vorliegenden Figuren die gleichen Bauteile mit dem gleichen Bezugszeichen versehen.

Es zeigen schematisch:
- Fig. 1: eine Ausführung der erfindungsgemässen Vorrichtung mit insgesamt vier Bearbeitungstrommeln;
- Fig. 2a: ein Übergaberad;
- Fig. 2b: einen Aufnahmegreifer in einem eingefahrenen Zustand;
- Fig. 2c: einen Aufnahmegreifer in einem ausgefahrenen Zustand;
- Fig. 2d: den Aufnahmegreifer aus Fig. 2c in Seitensicht;
- Fig. 3a: ein Grundaufbau einer Vorrichtung gemäss der vorliegenden Erfindung mit zwei Bearbeitungstrommeln und einem dazwischen angeordneten Übergaberad;
- Fig. 3b: schematisch einen Übergabebereich;
- Fig. 4: schematisch im Querschnitt durch die Rotationsachse einer Bearbeitungstrommel das Eingreifen eines Übergaberads, und
- Fig.5: einen Teilausschnitt aus einem Radius eines erfindungsgemässen Übergaberades.

### Ausführung der Erfindung

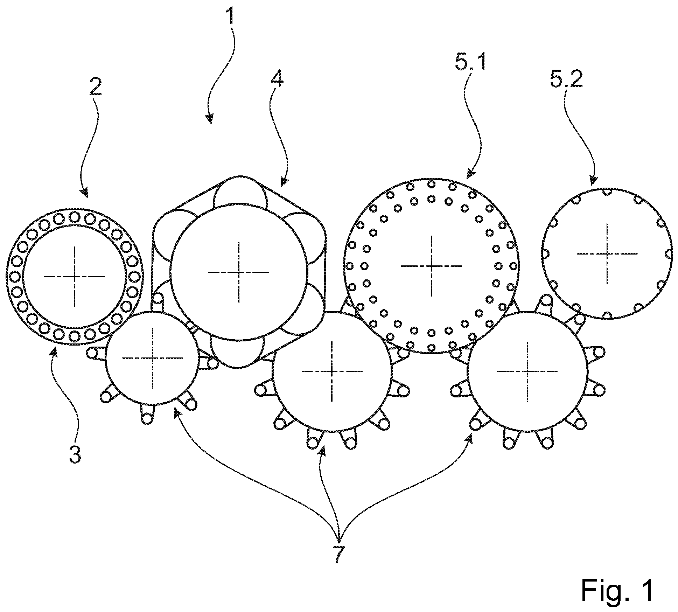

Die **Fig. 1** zeigt schematisch eine Vorrichtung 1 gemäss vorliegender Erfindung mit insgesamt vier Bearbeitungstrommeln 2, 4, 5.1, 5.2. Obschon dieses Beispiel mit vier Trommeln 2, 4, 5.1, 5.2 dargestellt wird, ist die Vorrichtung auch mit bloss zwei Trommeln 2, 4 bereits funktional. Die Bearbeitungstrommeln 2, 4, 5.1, 5.2 sind hintereinander angeordnet und vollführen je einen Bearbeitungsschritt in der Formung von Tampons.

Eine erste Bearbeitungstrommel 2 dient als Wickelstation. In die erste Bearbeitungstrommel 2 wird das bandförmige Material eingeführt, welches dann von der ersten Bearbeitungstrommel 2 zu Wickeln und dem ersten Vorformling gewickelt wird. Um das bandförmige Material zu wickeln, kann die erste Bearbeitungstrommel 2 mit einer Reihe an Gabeln ausgestattet sein, welche das bandförmige Material aufnehmen und mittels einer Drehbewegung um die eigene Längsachse zu einem Wickel verwickeln. Das bandförmige Material kann, wie eingangs geschildert, ein Laminat aus einem Vliesstoff und einer Watte sein. Dabei kann die Watte aus jedem Cellulose-Fasergewebe stammen, ist bevorzugt aber aus Viskose. Alternativ sind Baumwoll- oder Hanffasern denkbar. Der Vliesstoff umfasst bevorzugt ein thermoplastisches Copolymer.

Die Vorrichtung 1 weist eine Förderrichtung auf. Für das vorliegende Beispiel ist die Förderrichtung in der Fig. 1 von links nach rechts ausgelegt. Diese Förderrichtung wird von den Werkstücken im Betrieb durchlaufen.

Vorgelagerte und nicht gezeigte Bearbeitungsstationen können das entsprechende Laminat herstellen und das bandförmige Material bereits in der richtigen Grösse zugeschnitten in die erste Bearbeitungstrommel 2 einführen.

An die erste Bearbeitungstrommel 2 schliesst sich ein erstes Übergaberad 7 an. Dieses Übergaberad übernimmt die Wickel von der ersten Bearbeitungstrommel 2 und überträgt sie auf die zweite Bearbeitungstrommel 4. Im vorliegenden Beispiel ist die erste Bearbeitungstrommel 2 so ausgestattet, dass sie um die horizontale Rotationsachse im Uhrzeigersinn dreht. Die Werkzeuge, insbesondere Wickelgabeln, sind radial angeordnet. Das Übergaberad 7 ist so ausgestaltet, dass es im Gegenuhrzeigersinn um eine ebenfalls horizontal verlaufende Rotationsachse rotiert.

Die nachfolgende zweite Bearbeitungstrommel 4 ist eine Trommel zum Verpressen der Wickel und Vorformlinge zu zweiten Vorformlingen.

Die zweite Bearbeitungstrommel umfasst eine Mehrzahl von Presswerkzeugen. Die Presswerkzeuge können radial angeordnete Pressbacken sein. Insgesamt ist aber die Anzahl an Presswerkzeugen geringer als die Anzahl an Wickelwerkzeugen der ersten Bearbeitungstrommel. Das Übergaberad ist in der Lage ist, unterschiedliche Bahngeschwindigkeiten zwischen zwei Bearbeitungstrommeln 2, 4, 5.1, 5.2 zu kompensieren. Dies kann z. Bsp. dadurch gewährleistet werden, dass das Übergaberad eine entsprechende Führung umfasst, wie nachfolgend im Detail gezeigt.

Auf die zweite Bearbeitungstrommel 4, folgt eine dritte und vierte Bearbeitungstrommel 5.1, 5.2. Die erfindungsgemässe Vorrichtung 1 weist zwei Kopfform-/Glättungstrommeln 5.1, 5.2 auf. Auch diese Trommeln sind durch ein Übergaberad 7 jeweils mit der vorherigen Bearbeitungstrommel 4 verbunden, welches die entsprechende Bearbeitungstrommel 5.1 speist. Ebenso findet die Übergabe von der Kopfformtrommel 5.1 auf die Glättungstrommel 5.2 über eine Übergaberad 7 statt.

Die Übergaberäder 7 sind in so ausgestaltet, dass sie kontinuierlich um ihre zentralen Rotationsachsen drehbar sind. Die Rotationsachsen sind im vorliegenden Beispiel horizontal ausgerichtet und auf der Fig. 1 durch die gestrichelten Kreuze angedeutet. Im vorliegenden Ausführungsbeispiel rotieren im Betrieb die Übergaberäder im Gegenuhrzeigersinn, während die Bearbeitungstrommeln 2, 4, 5.1, 5.2 im Uhrzeigersinn rotieren. Selbsterklärend ist umgekehrter Aufbau möglich.

Die gesamte Anordnung kann an einem Maschinengestell (nicht gezeigt) montiert sein. Alternativ sind auch Werkbänke, oder die Montage in einem Werkraum denkbar. Ebenfalls denkbar ist die Integration in eine Vollautomatisierte Fertigungsstrasse mit vor- und nachgelagerter Bearbeitung.

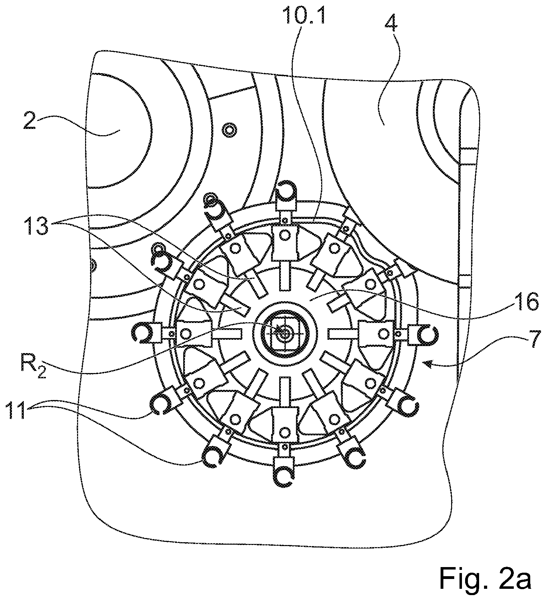

Die **Fig. 2a** zeigt schematisch ein erfindungsgemässes Übergaberad 7 in Detailansicht. Zur exemplarischen Darstellung ist das Übergaberad 7 zwischen zwei Bearbeitungstrommeln dargestellt. Das Übergaberad 7 ist rotierbar um eine zentrale Rotationsachse R2, welche horizontal ausgerichtet ist, gelagert. Dabei rotiert eine Drehscheibe 16 um die zentrale Rotationsachse R2. Mit der Drehscheibe 16 sind die Aufnahmegreifer 12 verbunden, welche über radial verschiebbare Führungsstangen 13 verfügen, welche in Führungsbuchsen gelagert sind. Die Führungsstangen 13 enden in Aufnahmenestern 11, welche ausgelegt sind, ein Werkstück, im vorliegenden Fall ein Vorformling oder einen Wickel, aufzunehmen.

Wie an der Figur erkennbar, sind die Bearbeitungstrommeln versetzt zueinander entlang ihrer Rotationsachsen angeordnet, sodass das Übergaberad 7 zwischen den beiden Bearbeitungstrommeln 2, 4 wirken kann.

Um die unterschiedlichen Bahngeschwindigkeiten der Bearbeitungstrommeln auf den Radien der Übergaberäder zu kompensieren, ist auf der Fig. 2a eine erste Führungskurve 10.1 ersichtlich, welche starr bzgl. der Drehscheibe 16 angeordnet ist.

Die Aufnahmegreifer 12 sind so ausgestaltet, dass sie mit der ersten Führungskurve 10.1 in Wirkverbindung stehen und bei der Rotation des Übergaberads 7 dem Kurvenverlauf dahingehend folgen, dass die Führungsstangen 13 zusätzlich zur radialen Rotation um die Rotationsachse des Übergaberads eine von der Führungskurve vorgegebene laterale Verschwenkung und craniale Verschiebung vornehmen können (hinsichtlich des Aufnahmenestes als Kopfende). Die Aufnahmegreifer 12 werden durch diese erste Führungskurve 10.1, wie durch eine Kulisse geführt.

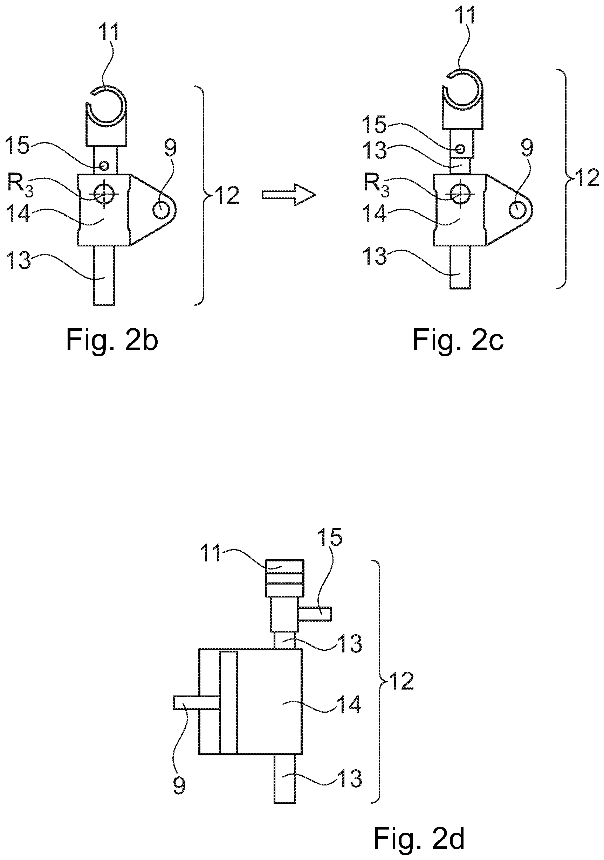

Um die Wirkungsweise der Aufnahmegreifer 12 im erfindungsgemässen Beispiel weiter zu erläutern, ist ein solcher noch schematisch in den **Fig. 2b,2c** und **2d** einzeln zeigt.

Der Aufnahmegreifer 12 umfasst geführte Führungsstangen 13, die mittels einer ein Linearlager aufweisenden Führungsbuchse 14entlang ihrer Längsachse verschiebbar gelagert sind.

Eine erste Nocke 15 ist an der Führungsstange 13 ausgebildet, um in eine Führungsnut der Führungskurve gemäss **Fig. 2a** einzugreifen. In der Darstellung der Fig. 2b und 2c, würde diese erste Nocke 15 sich in die dem Betrachter abgewandte Papierebene erstrecken.

Im vorliegenden Beispiel ist der Aufnahmegreifer 12 um eine Rotationsachse R3 an der Drehscheibe 16 rotierbar gelagert. Dies kann mittels eines Radiallagers bewerkstelligt werden. Im vorliegenden Beispiel ist das Radiallager durch die Drehscheibe gebildet, und durch einen Zapfen an der Rotationsachse R3 mit der Führungsbuchse wirkverbunden.

Die Führungsstange 13 endet kopfseitig mit einem Aufnahmenest 11, welche ein Lumen beschreibt, das zur Aufnahme des Werkstücks dient. Eine laterale Ausnehmung dient dazu, bei der Positionierung den horizontal in einen Übergabebereich hineinragenden Rückholfaden bei der Übergabe nicht zu berühren.

Die Fig. 2c zeigt eine Verschiebung der gelagerten Führungsstange 13 in der Längsachse des Aufnahmegreifers 12, wie sie durch ein Linearlager der Führungsbuchse 14 ermöglicht ist.

Die Führungsbuchse 14 weist eine zweite Nocke 9 auf der dem Betrachter zugewandten Papierebene auf. Diese zweite Nocke 9 kann mit einer zweiten Führungskurve in Wirkverbindung gebracht werden (in der Fig. 2a nicht gezeigt). Diese zweite Führungskurve dient als Kulisse zur Führung der Führungsbuchse 14 entlang einer Kurve. Dadurch kann sich das Aufnahmenest 11 in zwei Freiheitsgraden bei einer Rotation des Übergaberades 7 bewegen, einer Längsverschiebung in Längsrichtung und einer Rotation um die Rotationsachse R3. Da die beiden Nocken 9, 15 versetzt in Bezug zueinander angeordnet sind, unterstützt die Führung durch die zwei Führungskurven die Radialbewegung der Aufnahmegreifer auf ihren Kurven.

Die **Fig. 2d** zeigt den Aufnahmegreifer 12 aus der Fig. 2c nochmals um 90 Grad gedreht in Seitenansicht. Die Führungsstange 13 ist dabei leicht in radialer Richtung (bezüglich der Rotationsachse R2 des Übergaberades, in der Fig. 2d nicht gezeigt) verschoben. Die Führungsbuchse weist die Nocke 9 auf. Die Führungsstange weist die Nocke 15 auf. Am Aufnahmenest 11 ist in Aufsicht die laterale Ausnehmung erkennbar.

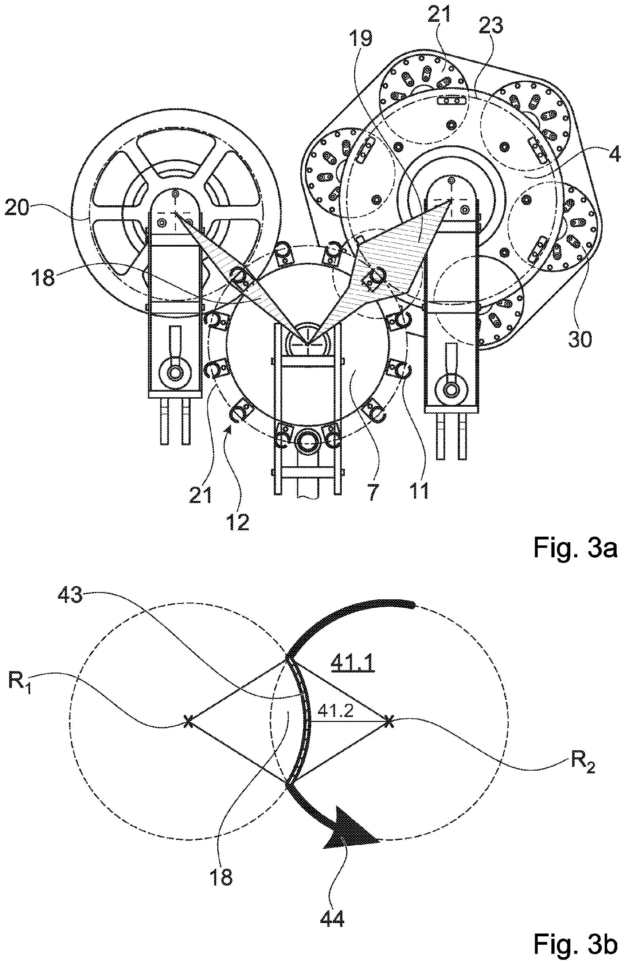

In der **Fig. 3a** ist eine Anordnung zur besseren Illustration des Übergaberads 7 mit zwei Bearbeitungstrommeln gezeigt. Eine erste Bearbeitungstrommel beschreibt mit den Bearbeitungswerkzeugen einen ersten Radius 20, während eine zweite Bearbeitungstrommel 4 mit ihren Werkzeugen einen zweiten Radius 23 beschreibt. Dabei beziehen sich diese Radien auf die entsprechende Lokalisation des Werkstücks im Werkzeug. Bei der zweiten Bearbeitungstrommel 4 handelt es sich um eine Bearbeitungstrommel 4 mit einer Mehrzahl an Tamponpressen 25, 30, welche radial um die Rotationsachse der Bearbeitungstrommel 4 angeordnet sind.

Die Rotationsachsen der Bearbeitungstrommeln 2, 4 sind schematisch durch ein zentrales Kreuz dargestellt. Entlang dieser Achsen kann eine Rotationswelle mit Rotationslager mit einem Rotationantrieb verbunden werden (nicht gezeigt), wobei ein Direktantrieb der einzelnen Bearbeitungstrommeln, oder ein einzelner Antrieb über ein Bandsystem beide Trommeln antreiben kann.

Die Presswerkzeuge 25, 30 sind mit mehreren radial angeordneten Presswerkzeugen, insbesondere mit Pressbacken, ausgestattet, welche einen in ihrem Mittelpunkt befindlichen Vorformling verpressen. Im vorliegenden Beispiel sind sechs Presswerkzeuge 25, 30 ausgebildet, welche Rotationssymmetrisch um die Rotationsachse angeordnet sind, um Unwuchten zu vermeiden.

Die Presswerkzeuge 25, 30 sind im vorliegenden Beispiel so ausgestaltet, dass sie einen Verpressungsschritt vom Zeitpunkt der Übernahme des Werkstücks, bis zur Übergabe an eine nächste Bearbeitungstrommel, oder an eine Entnahmeeinheit, durchführen. Dadurch lässt sich der Verpressungsschritt über einen Zeitraum durchführen, während das Werkstück, also der Vorformling, in Förderrichtung bewegt wird. Zeitgleich bereitet sich ein anderes Presswerkzeug auf die Übernahme eines folgenden ersten Vorformlings vor.

Der Prozess kann kontinuierlich laufen, ohne dass ein Verlust an Bearbeitungszeit während des Verpressens für den einzelnen Vorformling auftritt, was eine gute Bearbeitungsqualität erhält.

Das Übergaberad 7 ist ebenfalls an einer horizontalen Rotationsachse rotierbar gelagert und befindet sich zwischen den beiden Bearbeitungstrommeln. Das Übergaberad 7 weist insgesamt zwölf Aufnahmegreifer 12 auf, welche radial angeordnet sind und mit ihren Aufnahmenestern 11 ebenfalls einen Wirkradius 21 definieren. Im Betrieb bildet sich zwischen dem Wirkradius 21 des Übergaberads 7 und dem Wirkradius 20 der ersten Bearbeitungstrommel ein Übergabebereich 18. In einer Tangente zum Radius 20 der Bearbeitungstrommel innerhalb dieses Übergabebereichs 18 findet die Übergabe des Werkstücks von der ersten Bearbeitungstrommel zum Übergaberad 7 statt.

Entsprechend der geringeren Anzahl an Werkzeugen an der zweiten Bearbeitungstrommel 4 ist der Übergabebereich 19 zwischen dem Übergaberad 7 und der zweiten Bearbeitungstrommel 4 anders gestaltet.

Durch eine entsprechende Kurvenführung (in der Fig. 3 nicht gezeigt) kann der Aufnahmegreifer bei seiner Radialbewegung des Übergaberads optimal die entsprechenden Übergabebereiche 18, 19 abdecken.

So kann zum Beispiel mit Hilfe der Führungskurven eine gleiche Kurvengeschwindigkeit zwischen Aufnahmegreifer 12 und Bearbeitungswerkzeug während eines bestimmten Zeitfensters sichergestellt werden, in dem die Übergabe stattfinden kann.

Dieses Konzept ist in der Fig. 3b nochmals schematisch als Beispiel dargestellt. Ein erster Rotationsradius um die Rotationsachse R1 einer Bearbeitungstrommel ist links dargestellt. Dieser überlappt sich mit einem zweiten Rotationsradius Rotationsachse R2eines Übergaberades um die. Dadurch entsteht der Übergabebereich 18, in dem die Werkzeuge der Bearbeitungstrommel weiter ihrem Rotationsradius folgen, der Rotationsradius des Übergaberades aber einer Kurve 43 folgt, welche von der Rotationskurve 44 abweicht.

Dies ist durch die Freiheitsgrade der Aufnahmegreifer und die Führung entlang der Führungskurven ermöglicht, wie oben geläutert. So wird ein erster Kurvenradius 41.1 eines Aufnahmegreifers dynamisch an den Rotationsradius der Bearbeitungstrommel angepasst, und an einem zweiten Kurvenradius 41.2 in einer Tangente zum Rotationsradius der Bearbeitungstrommel das Werkstück übergeben.

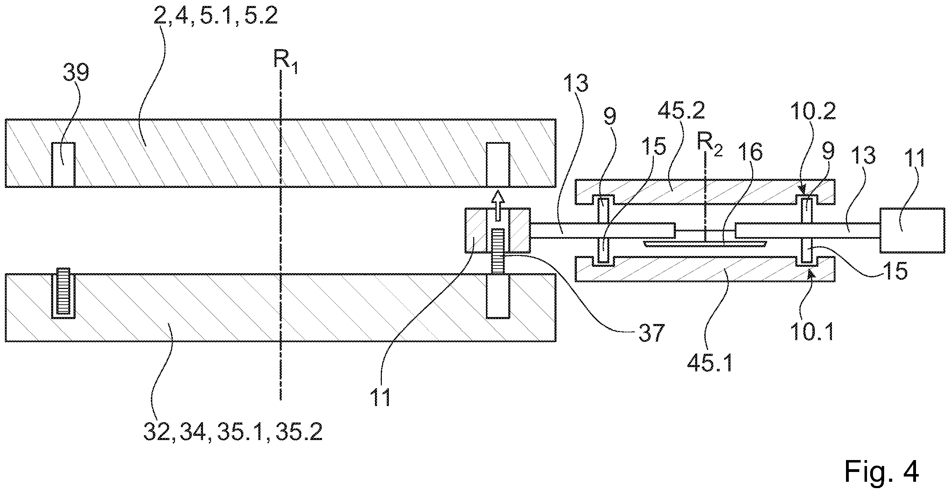

Die **Fig. 4** zeigt die Übergabe weiter im Detail am Beispiel einer beliebigen Bearbeitungstrommel 2, 4, 5.1, 5.2 welche eine zugehörige Ausstosstrommel 32, 34, 35.1, 35.2 aufweist, die um die gleiche Rotationsachse R1 drehbar gelagert ist.

Die Bearbeitungstrommel 2, 4, 5.1, 5.2 ist im Querschnitt gezeigt, wobei jeweils schematisch zwei Werkzeuge 39 an radial gegenüberliegenden Umkreisradien dargestellt sind. Diese Werkzeuge 39 dienen der Aufnahme des Werkstücks oder Vorformlings. Die Werkzeuge 39 können auch für einen bestimmten Bearbeitungsschritt verantwortlich sein. So kann ein Werkzeug einer Bearbeitungsstation zum Beispiel ein Wickelwerkzeug, als Presswerkzeug oder als Kopfform-/Glättwerkzeug ausgestaltet sein.

Die Bearbeitungstrommel 2, 4, 5.1, 5.2 definiert hinsichtlich der Ausstosstrommel 32, 34, 35.1, 35.2 einen Abstand, durch welcher ein Aufnahmegreifer eines Übergaberads einwirken kann in einen Übergabebereich, der sich zwischen der Bearbeitungstrommel und der Ausstosstrommel befindet, und zwar koaxial zwischen einem Ausstossstössel 37 und dem Werkzeug 39. Die Bearbeitungstrommeln 2, 4, 5.1, 5.2 sind in der Fig. 4 exemplarisch mit zugehörigen Ausstosstrommeln 32, 34, 35.1, 35.2 gezeigt. In der Praxis und einer konkreten Ausgestaltung einer erfindungsgemässen Vorrichtung kann eine Bearbeitungstrommel 2, 4, 5.1, 5.2 fest zugeordnete Ausstosstrommeln 32, 34, 35.1, 35.2 aufweisen, die an die strukturellen und funktionellen Eigenschaften der Bearbeitungstrommel 2, 4, 5.1, 5.2, z.B. hinsichtlich der Anzahl Werkzeuge, der Drehgeschwindigkeit, der Geometrie, angepasst ist. So kann zum Beispiel eine zur Verpressung der Vorformlinge vorgesehene zweite Bearbeitungstrommel 4 mit insgesamt sechs Werkzeugen, also Presswerkzeugen, eine entsprechend gestaltete Ausstosstrommel 34 aufweisen, mit sechs Ausstossstösseln 37 und entsprechender Kulisse zur Führung dieser Ausstossstössel.

Während der Übergabe ist ein Aufnahmenest 11 so in diesem Zwischenraum angeordnet, dass eine vom Aufnahmenest 11 definierte Aufnahmekammer koaxial zum Ausstossstössel 37 steht. Dieser Ausstossstössel 37 kann dreidimensional auf der Ausstosstrommel 32, 34, 35.1, 35.2 so geführt sein, dass er synchron im Übergabebereich mit der koaxialen Anordnung des Aufnahmenests 11 in den Übergabebereich derart hineinragt, dass er das Werkstück vom Aufnahmenest in Pfeilrichtung in das Werkzeug 39 hineinbefördert.

Um die Bahngeschwindigkeit zwischen dem Übergaberad und der Bearbeitungstrommel resp. Ausstosstrommel zum Zeitpunkt der Übergabe anzugleichen, ist das Übergaberad mit Nocken 9,15 versehen, welche je in eine entsprechende Nut einer Führungskurve 10.1, 10.2 wirken und durch welche die verschiebbar gelagerten Führungsstangen 13 dem Bewegungsverlauf der Kurve folgen. Dabei sind die Führungsstangen 13 über eine Drehscheibe 16 um eine Rotationsachse R2 drehbar gelagert. Die Führungskurven 10.1, 10.2 sind im vorliegenden Beispiel auf zwei parallel gegenüberliegend angeordneten Führungsscheiben 45.1, 45.2 als Nuten ausgebildet. Die Führungsscheiben 45.1, 45.2 sind gegenüber der Drehscheibe 16 starr. Die Führungskurven 10.1, 10.2 bilden so Kulissen, welche die Kurvenbewegung der Aufnahmegreifer steuern. Die Aufnahmegreifer sind zudem rotierbar auf der Drehscheibe 16 gelagert, so dass sie eine Schwenkbewegung senkrecht zur Papierebene vollführen können.

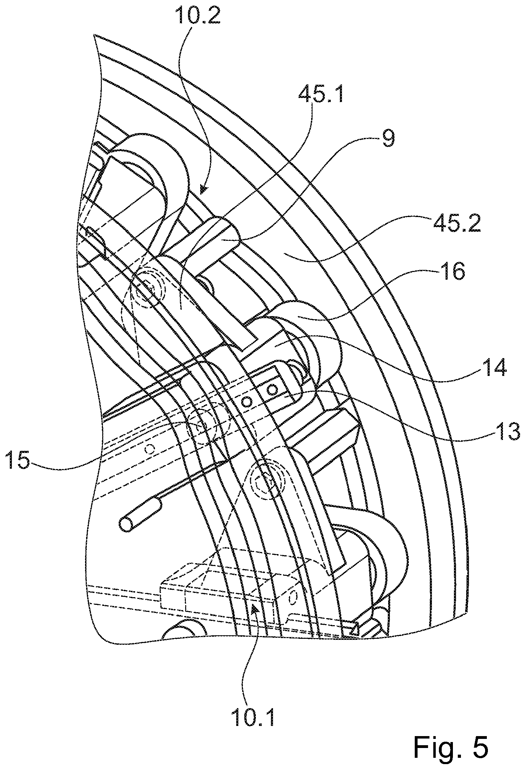

Die Fig.5 illustriert schematisch den Aufbau eines Übergaberades mit montierten Aufnahmegreifern in einer Detailansicht. Zwei Führungsschreiben 45.1, 45.2 sind parallel angeordnet, so dass sie einander eine Stirnseite zuwenden. Auf den jeweiligen Stirnseiten sind die Führungskurven 10.1, 10.2 als Nuten ausgebildet. In die Nuten der Führungskurven 10.1, 10.2 greifen jeweils Eingreifmittel 9, 15 der Aufnahmegreifer ein, wobei die Aufnahmegreifer in der vorliegenden Darstellung ohne Aufnahmenester dargestellt sind, um den Einblick in den Zwischenraum zwischen die Führungsscheiben 45.1, 45.2 nicht zu behindern.

Die Führungsbuchse 14 ist über eine zweite Nocke 9 als Eingreifmittel mit der abgewandten zweiten Führungskurve 10.2 wirkverbunden, so dass diese auf die Führungsbuchse 14 bei der rotatorischen Bewegung um die Rotationsachse R2 des Übergaberades wie mit einer Kulisse geführt ist. Die Führungsbuchse 14 ist weiterhin über ein Radiallager mit der Drehscheibe 16 schwenkbar gelagert. Die Drehscheibe 16 ist im vorliegenden Fall als Transportrad ausgebildet, welches radial angeordnete Lager aufweist zur Wirkverbindung mit den Führungsbuchsen 14.

Über die Führungsbuchse 14 ist die Führungsstange 13 linear gelagert. Die Führungsstange 13 weist eine Nocke 15 auf, welche auf analoge weise sich in die als Nut ausgebildete erste Führungskurve 10.1 erstreckt, und mit dieser eine Kulissen-Wirkverbindung eingeht.

An den Nocken 9, 15 können Laufräder angebracht sein, um die Reibung mit den Nuten der Führungskurven 10.1, 10.2 zu minimieren.

Mit der vorliegenden Erfindung ist eine Vorrichtung bereitgestellt, die eine kontinuierliche und skalierbare Tamponformung ermöglicht, welche eine Reihe von Vorteilen gegenüber den bekannten Systemen aufweist. Für einen Fachmann ergeben sich aus diesen konkreten Ausführungsbeispielen weitere vorteilhafte Ausführungsformen, welche mit weiteren Merkmalen aus der allgemeinen Beschreibung ergänzt werden können.

## Patentansprüche

1. **Vorrichtung** (1) zur Formung von Tampons, umfassend
a. eine Mehrzahl an **Bearbeitungstrommeln** (2, 4, 5.1, 5.2) zur Formung eines Tampons aus bandförmigem Material, und
**gekennzeichnet durch**
b. mindestens eine **Übergabestation (7),** zur kontinuierlichen Übergabe eines Vorformlings von einer ersten Bearbeitungstrommel (2, 4, 5.1, 5.2) an eine zweite Bearbeitungstrommel (4, 5.1, 5.2), und
wobei die Übergabestation (7) ein Übergaberad (7) zur kontinuierlichen Übergabe eines Vorformlings von einer ersten Bearbeitungstrommel (2, 4, 5.1, 5.2) an eine zweite Bearbeitungstrommel (4, 5.1, 5.2) ist.

2. Vorrichtung gemäss Anspruch 1, wobei das Übergaberad (7) ein rotierbar um eine Rotationsache R2 gelagertes Übergaberad (7), wobei die Rotationsache R2 parallel ist zu einer Rotationsachse R1 der Bearbeitungstrommeln (2, 4, 5.1, 5.2).

3. Vorrichtung (1) gemäss Anspruch 1 oder 2, wobei die Mehrzahl an Bearbeitungstrommeln (2, 4, 5.1, 5.2) eine **erste Bearbeitungstrommel** (2) zur Wicklung des bandförmigen Materials zu einem ersten Vorformling umfasst.

4. Vorrichtung (1) gemäss einem der Ansprüche 1 bis 3, wobei die Mehrzahl an Bearbeitungstrommeln (2, 4, 5.1, 5.2) **eine zweite Bearbeitungstrommel (4),** zur Pressung des ersten Vorformlings zu einem zweiten Vorformling umfasst.

5. Vorrichtung (1) gemäss einem der Ansprüche 1 bis 4, die Mehrzahl an Bearbeitungstrommeln (2, 4, 5.1, 5.2) mindestens **eine dritte Bearbeitungstrommel** (5.1, 5.2) zur Formung und/oder Glättung eines Tamponkopfes am zweiten Vorformling umfasst.

6. Vorrichtung (1) gemäss einem der Ansprüche 2 bis 5, wobei das Übergaberad (7) eine Mehrzahl an **Aufnahmegreifern** (12) umfasst, welche so ausgelegt sind, dass sie in **einen Übergabebereich** (18, 19) **eines Rotationsradius** (20, 23) mindestens einer der Bearbeitungstrommeln (2, 4, 5.1, 5.2) eingreifen.

7. Vorrichtung (1) gemäss Anspruch 6, wobei das Übergaberad (7) mindestens **eine Führungskurve** (10.1, 10.2) aufweist, der die **Aufnahmegreifer (12)** auf **einer Radialbewegung** so führt, dass bei einer Rotation des Übergaberads (7) relativ zu einer Bearbeitungstrommel (2, 4, 5.1, 5.2) ein Übergabebereich (18, 19) definiert wird, in welchem zumindest zeitweise **ein Aufnahmenest** (11) eines Aufnahmegreifers (12) im Wesentlichen koaxial **zu einem Bearbeitungswerkzeug** (39) einer Bearbeitungstrommel steht.

8. Vorrichtung (1) gemäss einem der Ansprüche 2 bis 7, wobei das Übergaberad (7) radial angeordnete **Aufnahmegreifer** (12) aufweist, und diese Aufnahmegreifer (12) umfassen:
a. ein **Aufnahmenest** (11) zur Aufnahme eines Tampons oder eines Vorformlings;
b. eine **Führungsstange** (13), an der das Aufnahmenest (11) angeordnet ist, und
c. eine **Führungsbuchse** (14), durch welche die Führungsstange (13) in ihrer Längsachse verschiebbar gelagert ist, und die an einer rotierbaren **Drehscheibe** (16) gelagert ist, insbesondere mittels eines Radiallagers gelagert ist.

9. Vorrichtung gemäss einem der Ansprüche 2 - 8, wobei das Übergaberad zwei parallel angeordnete Führungsscheiben (45.1, 45.2) umfasst, auf welcher je eine Führungskurve (10.1, 10.2) ausgestaltet ist, durch welche die Aufnahmegreifer (12) entlang einer Radialbewegung um die Rotationsachse R2 des Übergaberads dergestalt geführt sind, dass ein Wirkradius der Aufnahmegreifer zumindest zeitweise einem Rotationsradius (21, 23) eines Bearbeitungswerkzeugs folgt und eine Übergabe in einer Tangente zum Rotationsradius (21, 23) der Bearbeitungstrommel (2, 4, 5.1, 5.2) erfolgt.

10. Vorrichtung (1) gemäss einem der Ansprüche 1 bis 9, wobei die Bearbeitungstrommeln (2, 4. 5.1, 5.2) eine **Ausstosstrommel** (32, 34, 35.1, 35.2) umfassen, die um die gleiche Achse wie die zugehörige Bearbeitungstrommel (2, 4. 5.1, 5.2) rotierbar angeordnet ist.

11. Vorrichtung (1) gemäss Anspruch 10, wobei die Ausstosstrommel (32, 34, 35.1, 35.2) eine Mehrzahl an **Ausstossstösseln** (37) umfasst, welche derart auf einem Radius auf der Ausstosstrommel (32, 34, 35.1, 35.2) angeordnet sind, dass sie koaxial zu den Bearbeitungswerkzeugen der zugehörigen Bearbeitungstrommel angeordnet sind.

12. Verfahren zur Formung eines Tampons aus bandförmigem Material, insbesondere mit einer Vorrichtung gemäss einem der Ansprüche 1 bis 10, umfassend die Schritte:
a. **Bereitstellen** eines bandförmigen Materials;
b. schrittweises **Formen** des bandförmigen Materials über eine Mehrzahl von drehenden Bearbeitungstrommeln, insbesondere von kontinuierlich drehenden Bearbeitungstrommeln, wobei
eine **Übergabe** von einer ersten Bearbeitungstrommel zu einer zweiten Bearbeitungstrommel kontinuierlich mittels eines Übergaberades erfolgt.

13. Verfahren gemäss Anspruch 12, wobei das schrittweise Formen des bandförmigen Materials einen Wickelschritt, einen Pressschritt und einen Form- und/oder Glättungsschritt umfasst, und wobei jeder dieser Schritte auf einer bestimmten, kontinuierlich drehenden Bearbeitungstrommel erfolgt.

14. Verfahren gemäss Anspruch 13, wobei die Bearbeitungstrommeln bei jedem Schritt des schrittweisen Formens das Werkstück in eine Förderrichtung bewegen.

15. Verfahren gemäss einem der Ansprüche 11 bis 14, wobei das Übergaberad bei der Übergabe von einer ersten Bearbeitungstrommel zu einer zweiten Bearbeitungstrommel folgende Schritte vollführt:
a. **eingreifen** mit einem Aufnahmegreifer in einen Übergabebereich einer ersten Bearbeitungstrommel, so dass zumindest zeitweise ein Aufnahmenest des Aufnahmegreifers sich in einer koaxialen Anordnung zu einem Werkzeug befindet, und
b. **ausstossen** eines Werkstücks aus dem Werkzeug in das Aufnahmenest, oder aus dem Aufnahmenest in das Werkzeug, insbesondere durch **Verschieben** eines Ausstossstössels in Längsrichtung des Werkzeugs oder Aufnahmenests.

16. Verfahren gemäss einem der Ansprüche 11 bis 15, wobei der Ausstossstössel im Schritt b) auf einer Ausstosstrommel angeordnet ist, welche parallel zur entsprechenden Bearbeitungstrommel rotiert, so dass die Ausstossstössel jeweils einem entsprechenden Werkzeug zugeordnet sind.

17. Verfahren gemäss einem der Ansprüche 11 bis 16, wobei ein Aufnahmegreifer des Übergaberades bei der Rotation desselben über eine Führungskurve geführt ist, so dass in einem Übergabebereich zwischen der Bearbeitungstrommel, insbesondere einem Werkzeug der Bearbeitungstrommel, und einem Aufnahmenest die gleiche Bahngeschwindigkeit besteht.

18. Verwendung einer Übergabestation (7) mit radial umlaufend angeordneten Aufnahmegreifern (12), insbesondere eines Übergaberades (7), zur kontinuierlichen Übergabe eines Vorformlings von einer ersten Bearbeitungstrommel (2, 4, 5.1, 5.2) an eine zweite Bearbeitungstrommel (4, 5.1, 5.2) einer Vorrichtung gemäss Anspruch 1 zur Herstellung von Tampons oder Vorformlingen.

## Claims

1. **Apparatus** (1) for forming tampons, comprising
a. a plurality of **processing drums** (2, 4, 5.1, 5.2) for forming a tampon from band-form material, and
**characterized by**
b. at least one **transfer station (7)** for continuously transferring a preform from a first processing drum (2, 4, 5.1, 5.2) to a second processing drum (4, 5.1, 5.2), and
wherein the transfer station (7) is a transfer wheel (7) for continuously transferring a preform from a first processing drum (2, 4, 5.1, 5.2) to a second processing drum (4, 5.1, 5.2).

2. Apparatus (1) according to Claim 1, wherein the transfer station (7) is a transfer wheel (7) that is mounted so as to be rotatable about an axis of rotation R2, wherein the axis of rotation R2 is parallel to an axis of rotation R1 of the processing drums (2, 4, 5.1, 5.2).

3. Apparatus (1) according to Claim 1 or 2, wherein the plurality of processing drums (2, 4, 5.1, 5.2) comprises a **first processing drum** (2) for winding the band-form material to form a first preform.

4. Apparatus (1) according to one of Claims 1 to 3, wherein the plurality of processing drums (2, 4, 5.1, 5.2) comprises a **second processing drum (4)** for pressing the first preform to form a second preform.

5. Apparatus (1) according to one of Claims 1 to 4, wherein the plurality of processing drums (2, 4, 5.1, 5.2) comprises at least **one third processing drum** (5.1, 5.2) for forming and/or smoothing a tampon head on the second preform.

6. Apparatus (1) according to one of Claims 2 to 5, wherein the transfer wheel (7) comprises a plurality of **receiving grippers** (12), which are designed such that they engage in **a transfer region** (18, 19) **at a rotation radius** (20, 23) of at least one of the processing drums (2, 4, 5.1, 5.2).

7. Apparatus (1) according to Claim 6, wherein the transfer wheel (7) has at least **one guide curve** (10.1, 10.2) that guides the **receiving gripper (12)** in **a radial movement** such that, during a rotation of the transfer wheel (7) relative to a processing drum (2, 4, 5.1, 5.2), a transfer region (18, 19) is defined, in which, at least for a while, **a receiving nest** (11) of a receiving gripper (12) is substantially coaxial **with a processing tool** (39) of a processing drum.

8. Apparatus (1) according to one of Claims 2 to 7, wherein the transfer wheel (7) has radially arranged **receiving grippers** (12) and these receiving grippers (12) comprise:
a. a **receiving nest** (11) for receiving a tampon or a preform;
b. a **guide rod** (13) on which the receiving nest (11) is arranged; and
c. a **guide bushing** (14) by way of which the guide rod (13) is mounted so as to be displaceable along its longitudinal axis, and which is mounted on a rotatable **hub** (16), in particular is mounted by means of a radial bearing.

9. Apparatus according to one of Claims 2-8, wherein the transfer wheel comprises two guide discs (45.1, 45.2), on each of which there is formed a guide curve (10.1, 10.2), by way of which the receiving grippers (12) are guided in a radial movement about the axis of rotation R2 of the transfer wheel such that an effective radius of the receiving gripper follows a rotation radius (21, 23) of a processing tool at least for a while and transfer takes place at a tangent to the rotation radius (21, 23) of the processing drum (2, 4, 5.1, 5.2).

10. Apparatus (1) according to one of Claims 1 to 9, wherein the processing drums (2, 4, 5.1, 5.2) comprise an **ejection drum** (32, 34, 35.1, 35.2), which is arranged so as to be rotatable about the same axis as the associated processing drum (2, 4, 5.1, 5.2).

11. Apparatus (1) according to Claim 10, wherein the ejection drum (32, 34, 35.1, 35.2) comprises a plurality of **ejection push rods** (37) that are arranged at a radius on the ejection drum (32, 34, 35.1, 35.2) such that they are arranged coaxially with the processing tools of the associated processing drum.

12. Method for forming a tampon from band-form material, in particular using an apparatus according to one of Claims 1 to 10, comprising the steps of:
a. **providing** a band-form material;
b. incrementally **forming** the band-form material via a plurality of rotating processing drums, in particular continuously rotating processing drums, wherein
**a transfer** from a first processing drum to a second processing drum takes place continuously by means of a transfer wheel.

13. Method according to Claim 12, wherein the incremental forming of the band-form material comprises a winding step, a pressing step and a forming and/or smoothing step, and wherein each of these steps takes place on a particular, continuously rotating processing drum.

14. Method according to Claim 13, wherein the processing drums move the workpiece in a conveying direction in each incremental forming step.

15. Method according to one of Claims 11 to 14, wherein, during the transfer from a first processing drum to a second processing drum, the transfer wheel executes the following steps of:
a. **engaging** with a receiving gripper in a transfer region of a first processing drum, such that a receiving nest of the receiving gripper is located at least for a while in a coaxial arrangement with a tool, and
b. **ejecting** a workpiece from the tool into the receiving nest, or from the receiving nest into the tool, in particular by **displacing** an ejection push rod in the longitudinal direction of the tool or receiving nest.

16. Method according to one of Claims 11 to 15, wherein, in step b), the ejection push rod is arranged on an ejection drum, which rotates parallel to the corresponding processing drum, such that the ejection push rods are each assigned to a corresponding tool.

17. Method according to one of Claims 11 to 16, wherein a receiving gripper of the transfer wheel is guided, during the rotation of the latter, over a guide curve, such that there is the same speed in a transfer region between the processing drum, in particular a tool of the processing drum, and receiving nest.

18. Use of a transfer station (7) having radially circumferentially arranged receiving grippers (12), in particular of a transfer wheel (7), for the continuous transfer of a preform from a first processing drum (2, 4, 5.1, 5.2) to a second processing drum (4, 5.1, 5.2) of an apparatus according to claim 1 for producing tampons or preforms.

## Revendications

1. Dispositif (1) permettant de former des tampons, comprenant
a) une pluralité de tambours de traitement (2, 4, 5.1, 5.2) pour former un tampon à partir d'un matériau en forme de bande, et
**caractérisé par**
b) au moins un poste de transfert (7) pour le transfert continu d'une ébauche d'un premier tambour de traitement (2, 4, 5.1, 5.2) à un deuxième tambour de traitement (4, 5.1, 5.2), et
dans lequel le poste de transfert (7) est une roue de transfert (7) pour le transfert continu d'une ébauche d'un premier tambour de traitement (2, 4, 5.1, 5.2) à un deuxième tambour de traitement (4, 5.1, 5.2).

2. Dispositif selon la revendication 1, dans lequel la roue de transfert (7) est une roue de transfert (7) montée en rotation autour d'un axe de rotation R2, l'axe de rotation R2 étant parallèle à un axe de rotation R1 des tambours de traitement (2, 4, 5.1, 5.2).

3. Dispositif (1) selon la revendication 1 ou 2, dans lequel la pluralité de tambours de traitement (2, 4, 5.1, 5.2) comprend un premier tambour de traitement (2) pour enrouler le matériau en forme de bande en une première ébauche.

4. Dispositif (1) selon l'une quelconque des revendications 1 à 3, dans lequel la pluralité de tambours de traitement (2, 4, 5.1, 5.2) comprend un deuxième tambour de traitement (4) pour presser la première ébauche en une deuxième ébauche.

5. Dispositif (1) selon l'une quelconque des revendications 1 à 4, dans lequel la pluralité de tambours de traitement (2, 4, 5.1, 5.2) comprend au moins un troisième tambour de traitement (5.1, 5.2) pour former et/ou lisser une tête de tampon sur la deuxième ébauche.

6. Dispositif (1) selon l'une quelconque des revendications 2 à 5, dans lequel la roue de transfert (7) comprend une pluralité de griffes de réception (12) qui sont conçues de telle sorte qu'elles s'engagent dans une zone de transfert (18, 19) d'un rayon de rotation (20, 23) d'au moins l'un des tambours de traitement (2, 4, 5.1, 5.2).

7. Dispositif (1) selon la revendication 6, dans lequel la roue de transfert (7) présente au moins une came de guidage (10.1, 10.2) qui guide les griffes de réception (12) par un mouvement radial de façon à définir en cas de rotation de la roue de transfert (7) par rapport à un tambour de traitement (2, 4, 5.1, 5.2) une zone de transfert (18, 19) dans laquelle une cavité de réception (11) d'une griffe de réception (12) se trouve au moins temporairement de manière substantiellement coaxiale par rapport à un outil de traitement (39) d'un tambour de traitement.

8. Dispositif (1) selon l'une quelconque des revendications 2 à 7, dans lequel la roue de transfert (7) présente des griffes de réception (12) disposées radialement, et lesdites griffes de réception (12) comprennent :
a) une cavité de réception (11) pour recevoir un tampon ou une ébauche ;
b) une tige de guidage (13) sur laquelle est disposée la cavité de réception (11), et
c) une douille de guidage (14) par laquelle la tige de guidage (13) est montée coulissante au niveau de son axe longitudinal et qui est montée en rotation au niveau d'un disque tournant rotatif (16), en particulier au moyen d'un roulement radial.

9. Dispositif selon l'une quelconque des revendications 2 à 8, dans lequel la roue de transfert comprend deux disques de guidage (45.1, 45.2) disposés en parallèle, sur lesquels est configurée respectivement une came de guidage (10.1, 10.2) qui guide les griffes de réception (12) le long d'un mouvement radial autour de l'axe de rotation R2 de la roue de transfert de telle sorte qu'un rayon d'action des griffes de réception suit au moins temporairement un rayon de rotation (21, 23) d'un outil de traitement et un transfert est effectué sur une tangente au rayon de rotation (21, 23) du tambour de traitement (2, 4, 5.1, 5.2).

10. Dispositif (1) selon l'une quelconque des revendications 1 à 9, dans lequel les tambours de traitement (2, 4. 5.1, 5.2) comprennent un tambour d'éjection (32, 34, 35.1, 35.2) qui est disposé en rotation autour du même axe que le tambour de traitement associé (2, 4. 5.1, 5.2).

11. Dispositif (1) selon la revendication 10, dans lequel le tambour d'éjection (32, 34, 35.1, 35.2) comprend une pluralité de poussoirs d'éjection (37) qui sont disposé sur un rayon sur le tambour d'éjection (32, 34, 35.1, 35.2) de façon à être coaxiaux par rapport aux outils de traitement du tambour de traitement associé.

12. Procédé permettant de former un tampon à partir d'un matériau en forme de bande, comprenant en particulier un dispositif selon l'une quelconque des revendications 1 à 10, comprenant les étapes consistant à :
a) fournir un matériau en forme de bande ;
b) former progressivement le matériau en forme de bande par l'intermédiaire d'une pluralité de tambours de traitement tournants, en particulier de tambours de traitement tournant en continu, dans lequel
un transfert d'un premier tambour de traitement à un deuxième tambour de traitement est effectué en continu au moyen d'une roue de transfert.

13. Procédé selon la revendication 12, dans lequel la formation progressive du matériau en forme de bande comprend une étape d'enroulement, une étape de pressage et une étape de formation et/ou de lissage, et dans lequel chacune de ces étapes est effectuée sur un tambour de traitement spécifique, tournant en continu.

14. Procédé selon la revendication 13, dans lequel les tambours de traitement déplacent la pièce à travailler dans un sens de la marche à chaque étape de la formation progressive.

15. Procédé selon l'une quelconque des revendications 11 à 14, dans lequel lors du transfert d'un premier tambour de traitement à un deuxième tambour de traitement, la roue de transfert effectue les étapes suivantes consistant à :
a) venir en prise avec une griffe de réception dans une zone de transfert d'un premier tambour de traitement de sorte qu'au moins temporairement une cavité de réception de la griffe de réception se trouve dans un agencement coaxial par rapport à un outil, et
b) éjecter une pièce à travailler de l'outil à la cavité de réception ou de la cavité de réception à l'outil, en particulier en déplaçant un poussoir d'éjection dans la direction longitudinale de l'outil ou de la cavité de réception.

16. Procédé selon l'une quelconque des revendications 11 à 15, dans lequel le poussoir d'éjection à l'étape b) est disposé sur un tambour d'éjection qui tourne en parallèle au tambour de traitement correspondant de sorte que les poussoirs d'éjection sont respectivement associés à un outil correspondant.

17. Procédé selon l'une quelconque des revendications 11 à 16, dans lequel une griffe de réception de la roue de transfert est guidée lors de la rotation de celle-ci par l'intermédiaire d'une came de guidage de sorte que la vitesse de bande est identique dans une zone de transfert entre le tambour de traitement, en particulier un outil du tambour de traitement, et une cavité de réception.

18. Utilisation d'un poste de transfert (7) pourvu de griffes de réception (12) disposées de manière radialement périphérique, en particulier d'une roue de transfert (7), pour transférer en continu une ébauche d'un premier tambour de traitement (2, 4, 5.1, 5.2) à un deuxième tambour de traitement (4, 5.1, 5.2) d'un dispositif selon la revendication 1 pour fabriquer des tampons ou des ébauches.
